# EUROPÄISCHE PATENTANMELDUNG

(11) **EP 2 108 388 A1**
(43) Veröffentlichungstag der Anmeldung: **14.10.2009**
(21) Anmeldenummer: 09004319.1
(22) Anmeldetag: 26.03.2009
(51) Int. Cl.: A61L 27/54, A01N 59/16, C08J 3/00, C08L 75/04, C09D 175/00, C08G 18/00

(54) **Wässrige silberhaltige nichtionische Polyurethandispersionen**

(30) Priorität: 08.04.2008 EP 08154209
(71) Anmelder: Bayer MaterialScience AG, 51368 Leverkusen (DE)
(72) Erfinder: Köcher, Jürgen, Dr., 40764 Langenfeld (DE); Eiden, Stefanie, Dr., 51371 Leverkusen (DE); Mayer-Bartschmid, Anke, Dr., 42489 Wülfrath (DE); Knezevic, Igor, Dr., 40627 Düsseldorf (DE)

(57) **Zusammenfassung**

Die vorliegende Erfindung betrifft eine wässrige Dispersion, umfassend mindestens einen nichtionisch stabilisierten Polyurethanharnstoff und mindestens einen silberhaltigen Bestandteil.

## Beschreibung

Die vorliegende Erfindung betrifft eine wässrige silberhaltige nichtionische Polyurethandispersion. Weiterer Gegenstand der vorliegenden Erfindung ist ein Verfahren zur Herstellung der wässrigen silberhaltigen nichtionischen Polyurethandispersion sowie deren Verwendung zur Herstellung von antibakteriellen (antimikrobiellen) Beschichtungen.

Kunststoff- und Metallgegenstände werden im medizinischen Bereich sehr häufig verwendet. Beispiele für derartige Materialien sind Implantate, Kanülen oder Katheter. Problematisch bei der Verwendung dieser Produkte ist das leichte Besiedeln der Oberflächen dieser Materialien mit Keimen. Die Folge der Verwendung eines mit Bakterien besiedelten Gegenstandes, wie eines Implantates, einer Kanüle oder eines Katheters, sind oft Infektionen durch Bildung eines Biofilms. Besonders gravierend sind solche Infektionen im Bereich von zentralvenösen Kathetern sowie im urologischen Bereich, wo Katheter verwendet werden.

Es wurden in der Vergangenheit bisher zahlreiche Versuche unternommen, die Besiedlung von Oberflächen mit Bakterien und somit Infektionen zu verhindern. Häufig wurde versucht, die Oberfläche von medizinischen Implantaten oder Kathetern mit Antibiotika zu imprägnieren. Dabei muss allerdings mit der Bildung und Selektion von resistenten Bakterien gerechnet werden.

Ein weiterer Ansatz zur Verhinderung von Infektionen bei der Verwendung von Implantaten oder Kathetern ist die Verwendung von Metallen oder Metalllegierungen, z. B. bei Kathetern.

Von besonderer Bedeutung ist hierbei die antibakterielle Wirkung von Silber. Silber und Silbersalze sind schon seit vielen Jahren als antimikrobiell wirksame Substanzen bekannt. Die antimikrobielle Wirkung von Oberflächen, die Silber enthalten, beruht auf der Freisetzung von Silberionen. Der Vorteil von Silber besteht in seiner hohen Toxizität gegenüber Bakterien schon in sehr niedrigen Konzentrationen. Hardes et al., Biomaterials 28 (2007) 2869-2875, berichten von einer bakteriziden Aktivität von Silber bis hinunter zu einer Konzentration von 35 ppb. Demgegenüber ist Silber auch bei einer deutlich höheren Konzentration gegenüber Säugetierzellen noch nicht toxisch. Ein weiterer Vorteil ist die geringe Neigung der Bakterien zur Bildung von Resistenzen gegenüber Silber.

Verschiedene Ansätze zur Ausrüstung medizinischer Geräte mit Silber, wie beispielsweise Kathetern, sind in der Literatur beschrieben. Ein Ansatz ist die Verwendung von metallischem Silber auf Katheteroberflächen. So ist beispielsweise aus der US 3,800,087 ein Verfahren zum Metallisieren von Oberflächen bekannt, welches gemäß der DE 43 28 999 auch an medizinischen Geräten, wie beispielsweise einem Katheter, angewendet werden kann. Nachteilig ist dabei, dass das Silber bei den Belastungen des Katheters schlecht haftet, beispielsweise bei Lagerung in Körperflüssigkeiten wie Urin, durch Reibung beim Einführen und Herausziehen aus dem Körper oder durch wiederholtes Biegen des Katheters.

Metallische Beschichtungen von medizinischen Geräten haben jedoch nicht nur den Nachteil der schlechten Haftung auf dem Kathetermaterial, sondern auch, dass ein Aufbringen auf den Innenseiten des Katheters nicht oder nur sehr aufwändig möglich ist.

Eine Verbesserung der Haftung der Silberschicht auf einem Katheterkunststoff beschreibt die bereits erwähnte DE 43 28 999 A, indem zwischen Kunststoff und Silberschicht besser haftende Metallschichten aufgebracht werden. Aufgebracht wird das Silber bei den beschriebenen Produkten durch Aufdampfen in einer Vakuumkammer, durch Sputtern oder auch durch lonenimplantation. Diese Verfahren sind sehr umständlich und kostspielig. Nachteilig ist auch, dass die Menge an aufgedampftem elementarem Silber relativ hoch ist, aber nur sehr geringe Mengen aktiver Silberionen an die umgebende Flüssigkeit abgegeben werden. Weiterhin kann man mit diesen Verfahren nur die Außenseite eines Implantates oder eines Katheters beschichten. Es ist aber bekannt, dass sich auch an der Innenseite eines Katheters leicht Bakterien anhaften, was zur Biofilmbildung und Infektion des Patienten führt.

Viele Anwendungen beschäftigen sich mit der Verwendung von Silbersalzen in antimikrobiellen Beschichtungen, die auf medizinische Implantate oder Katheter aufgebracht werden. Gegenüber metallischem Silber haben Silbersalze den Nachteil, dass in der imprägnierten Schicht neben dem wirksamen Silber noch Anionen enthalten sind, die unter Umständen toxisch sein können, wie beispielsweise Nitrat im Silbernitrat. Ein weiteres Problem ist die Freisetzungsrate von Silberionen aus Silbersalzen. Manche Silbersalze wie Silbernitrat sind sehr gut wasserlöslich und werden deshalb eventuell zu schnell aus der Oberflächenbeschichtung in das umliegende Medium abgegeben. Andere Silbersalze wie Silberchlorid sind so schwerlöslich, dass Silberionen unter Umständen zu langsam in die Flüssigkeit abgegeben werden.

So betrifft US 6,716,895 B1 antimikrobielle Zusammensetzungen, die als einen Bestandteil ein hydrophiles Polymer umfassen, welches unter anderem ausgewählt sein kann aus Polyetherpolyurethanen, Polyesterpolyurethanen und Polyurethanharnstoffen. Die antimikrobielle Beschichtung wird durch oligodynamische Salze, wie beispielsweise durch Verwendung von Silbersalzen, erreicht. Die Zusammensetzung wird zur Beschichtung von medizinischen Geräten verwendet. Nachteilig an dieser Beschichtung ist neben der bereits erwähnten Verwendung von Silbersalzen auch, dass sie ausgehend von einer Lösung der polymeren Bestandteile hergestellt wird, so dass es sich häufig nicht verhindern lässt, dass Rückstände von toxischen Lösemitteln nach der Implantation von medizinischen Geräten, welche mit dieser Beschichtung versehen sind, in den menschlichen Körper gelangen.

Weitere Veröffentlichungen, wie beispielsweise die WO 2004/017738 A, die WO 2001/043788 A und die US 2004/0116551 A beschreiben ein Konzept, durch Kombination verschiedener Silbersalze zu einer silberhaltigen Beschichtung zu kommen, die kontinuierlich Silberionen freisetzt. Die verschiedenen Silbersalze werden mit unterschiedlichen Polymeren, beispielsweise Polyurethanen, gemischt, und die Kombination von Silbersalzen verschiedener Wasserlöslichkeit ist so aufeinander abgestimmt, dass es über den gesamten Zeitraum der Nutzung des beschichteten Gerätes zu einer konstanten Silberfreisetzung kommt. Diese Verfahren sind durch die Verwendung mehrerer Silbersalze und mehrerer Polymere umständlich.

Weitere Verfahren unter Verwendung von Silberionen beschreiben die WO 2001/037670 A und US 2003/0147960 A. WO 2001/037670 A betrifft dabei eine antimikrobielle Formulierung, die Silberionen in Zeolithen komplexiert. US 2003/0147960 A beschreibt Beschichtungen, in denen Silberionen in einer Mischung aus hydrophilen und hydrophoben Polymeren gebunden sind.

Die beschriebenen Verfahren unter Verwendung von Silbersalzen weisen die zuvor erwähnten Nachteile auf und sind darüber hinaus von ihrer Durchführung her kompliziert und daher vom Aufwand der Herstellung teuer, so dass weiterhin ein Bedarf an verbesserten silberhaltigen Beschichtungen bezüglich des Herstellungsprozesses und der Wirksamkeit besteht.

Eine interessante Möglichkeit zur antimikrobiellen Ausrüstung von Kunststoffen ist die Verwendung von nanokristallinen Silberteilchen. Der Vorteil zum Beschichten mit metallischem Silber liegt in der im Verhältnis zum Volumen viel größeren Oberfläche des nanokristallinen Silbers, was zu einer erhöhten Freisetzung von Silberionen im Vergleich zu einer metallischen Silberbeschichtung führt.

Furno et al., Journal of Antimicrobial Chemotherapy 2004, 54, S. 1019-1024 beschreiben ein Verfahren, welches unter Verwendung von überkritischem Kohlendioxid nanokristallines Silber in Silikonoberflächen imprägniert. Dieses Verfahren ist aufgrund des komplizierten Imprägnierprozesses und der zwingenden Verwendung von überkritischem Kohlendioxid teuer und nicht leicht anwendbar.

Ferner sind verschiedene Verfahren bekannt, um nanokristallines Silber in Kunststoffe einzubauen. So beschreiben die WO 01/09229 A1, die WO 2004/024205 A1, die EP 0 711 113 A und Münstedt et al., Advanced Engineering Materials 2000, 2(6), Seiten 380 bis 386 den Einbau von nanokristallinem Silber in thermoplastische Polyurethane. Dabei werden Pellets aus einem handelsüblichen thermoplastischen Polyurethan in Lösung mit kolloidalem Silber durch Tränkung versetzt. Zur Erhöhung der antimikrobiellen Wirksamkeit erwähnen WO 2004/024205 A1 und DE 103 51 611 A1 zusätzlich, dass Bariumsulfat als Additiv verwendet werden kann. Aus den dotierten Polyurethanpellets werden dann durch Extrusion die entsprechenden Produkte wie Katheter hergestellt. Diese in den Veröffentlichungen beschriebene Vorgehensweise ist nachteilig, da die Menge an Silber, welche an den Polyurethanpellets nach dem Eintauchen verbleibt, nicht konstant ist bzw. nicht vorbestimmt werden kann. Der effektive Silbergehalt der resultierenden Produkte muss daher abschließend, d.h. nach Herstellung der Endprodukte, bestimmt werden. Demgegenüber ist ein Vorgehensweise, mit welcher die effektive Silbermenge, welche in dem resultierenden Endprodukt vorgesehen sein soll, genau eingestellt wird, aus diesen Veröffentlichungen nicht bekannt.

Ein ähnliches Verfahren beschreibt die EP 0 433 961 A. Auch hier wird eine Mischung aus einem thermoplastischen Polyurethan (Pellethan), Silberpulver und Bariumsulfat gemischt und extrudiert.

Ein Nachteil an diesen Verfahren ist darüber hinaus der relativ große Bedarf an Silber, welches im gesamten Kunststoffkörper verteilt wird. Dieses Verfahren ist daher teuer und durch den Einbau des kolloidalen Silbers in der ganzen Kunststoffmatrix ist die Freisetzung des Silbers für eine ausreichende Wirksamkeit in Einzelfällen zu langsam. Die Verbesserung der Silberfreisetzung durch Zugabe von Bariumsulfat bedeutet einen weiteren kostenintensiven Arbeitsschritt.

Eine Beschichtungslösung aus einem thermoplastischen Polyurethan mit nanokristallinem Silber in einem organischen Lösemittel zur Herstellung von Gefäßprothesen beschreibt die WO 2006/032497 A. Die Struktur des Polyurethans wird nicht weiter spezifiziert, aber aufgrund der Beanspruchung von thermoplastischen Kunststoffen ist von der Verwendung von harnstofffreien Polyurethanen auszugehen. Die antibakterielle Wirkung wurde über das Wachstum adhärierter *Staphylococcus epidermidis-Zellen* auf der Prüfkörperoberfläche im Vergleich zu einer Kontrolle bestimmt. Die detektierte antibakterielle Wirkung der silberhaltigen Beschichtungen ist allerdings als schwach zu bewerten, da nur eine Wachstumsverzögerung von maximal 33,2 h (ausgehend von einem bestimmten Schwellenwachstum) gegenüber der Kontrolloberfläche festgestellt wurde. Für längere Anwendungen als Implantat oder Katheter ist diese Beschichtungsformulierung somit nicht geeignet.

Ein weiteres Problem ist die Verwendung von Lösemitteln für die Herstellung von Beschichtungslösungen. WO 2006/032497 A1 beschreibt ein antimikrobielles Implantat mit einer flexiblen porösen Struktur aus einem bioverträglichen Kunststoff als Vliesstruktur. Dabei wird unter anderem eine Lösung von einem thermoplastischen Polyurethan in Chloroform verwendet. Chloroform ist als sehr toxisches Lösemittel bekannt. Bei Beschichtungen von Medizinprodukten, die im menschlichen Körper implantiert werden, besteht die Gefahr von Rückständen dieses toxischen Lösemittels nach Implantation in den menschlichen Körper.

Ein weiterer Nachteil von kolloidalem Silber in organischen Beschichtungslösungen ist die oft geringe Stabilität der Silber-Nanoteilchen. Es kann in organischen Lösungen zu Aggregaten von Silberteilchen kommen, so dass man keine reproduzierbare Silberaktivität einstellen kann. Eine mit kolloidalem Silber versetzte organische Lösung sollte daher möglichst bald nach der Herstellung zu fertigen Beschichtungen verarbeitet werden, um eine von Batch zu Batch konsistente Silberaktivität zu gewährleisten. Diese Vorgehensweise ist aber manchmal aufgrund der betrieblichen Praxis nicht möglich.

Daher ist eine wässrige Polyurethanbeschichtung mit enthaltenem kolloidal verteiltem Silber als antimikrobielle Beschichtung wünschenswert.

US 2006/045899 beschreibt antimikrobielle Formulierungen unter Zuhilfenahme von wässrigen Polyurethansystemen. Die antimikrobielle Formulierung ist eine Mischung verschiedener Materialien, was für die Herstellung dieser Produkte schwierig ist. Die Natur der wässrigen Polyurethansysteme wird nicht genau beschrieben, außer dass es sich um kationisch oder anionisch stabilisierte Dispersionen handelt.

CN 1760294 erwähnt ebenfalls anionische Polyurethandispersionen mit Siberpulver mit einer Partikelgröße von 0,2 bis 10 µm. Nach dem Stand der Technik sind solche Partikelgrößen für eine hohe antimikrobielle Wirksamkeit nicht ausreichend.

C.-W. Chou et al., Polymer Degradation and Stability 91 (2006), 1017-1024 beschreiben Sulfonat-modifizierte Dispersionen von Polyetherpolyurethanen, in die sehr kleine Mengen (0,00151 bis 0,0113 Gew.-%) kolloidales Silber eingebaut werden. Ziel dieser Arbeiten war die Verbesserung der thermischen und mechanischen Eigenschaften des eingesetzten Polyurethans. Eine antimikrobielle Wirkung wurde nicht untersucht und ist aufgrund der sehr geringen Mengen an Silber unwahrscheinlich.

Ausgehend von diesem Stand der Technik stellt sich die vorliegende Erfindung die Aufgabe, eine Zusammensetzung bereitzustellen, welche die zuvor genannten Nachteile nicht aufweist. Insbesondere soll die Zusammensetzung zu Beschichtungen führen, welche unter toxikologischen Gesichtspunkten unkritisch sind und das antimikrobiell wirkende Mittel schnell und dauerhaft freisetzen. Die Zusammensetzung soll dabei vorzugsweise so ausgebildet sein, dass sie das antimikrobiell wirkende Mittel in einer vorgegebenen Menge umfasst, so dass beispielsweise unterschiedliche Anwendungsgebiete der Zusammensetzung durch Variation der Menge des antimikrobiell wirkenden Mittels erschlossen werden können.

Diese Aufgabe wird durch die Bereitstellung von einer wässrigen Dispersion gelöst, welche mindestens einen nichtionisch stabilisierten Polyurethanharnstoff und mindestens einen silberhaltigen Bestandteil umfasst.

Erfindungsgemäß wurde herausgefunden, dass Beschichtungen aus Polyurethanharnstoffen, die in Wasser dispergiert sind und die einen silberhaltigen Bestandteil umfassen, eine wirkungsvolle Silberfreisetzung zeigen, wenn die Polyurethanharnstoffdispersion nichtionisch stabilisiert ist. Entsprechende erfindungsgemäße Versuche sowie entsprechende Vergleichsversuche, welche diese Erkenntnis stützen, sind weiter unten beschrieben.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 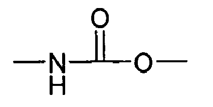 und
(b) mindestens eine Harnstoffgruppen enthaltende Wiederholungseinheit 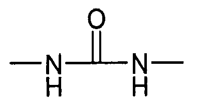
aufweisen.

Die erfindungsgemäßen Zusammensetzungen basieren auf Polyurethanharnstoffen, welche im Wesentlichen keine ionische Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Gruppen" wird im Rahmen der vorliegenden Erfindung verstanden, dass der Polyurethanharnstoff ionische Gruppen mit einem Anteil von im Allgemeinen höchstens 2,50 Gew.-%, insbesondere höchstens 2,00 Gew.-%, vorzugsweise höchstens 1,50 Gew.-%, besonders bevorzugt höchstens 1,00 Gew.-%, speziell höchstens 0,50 Gew.-%, noch spezieller keine ionische Gruppen aufweist. Damit ist insbesondere bevorzugt, dass der Polyurethanharnstoff keine ionische Gruppen aufweist.

Die erfindungsgemäß in der Zusammensetzung vorgesehenen Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man leicht anvernetzte Systeme, die eine Makropolyolkomponente als Aufbaukomponente, im Allgemeinen ausgewählt aus der Gruppe, bestehend aus einem Polyetherpolyol, einem Polycarbonatpolyol, einem Polyesterpolyol und Mischungen davon, umfassen, die eine mittlere Funktionalität von vorzugsweise 1,7 bis 2,3, insbesondere 1,8 bis 2,2, besonders bevorzugt 1,9 bis 2,1, aufweisen.

Sollten Mischungen von Makropolyolen und gegebenenfalls Polyole in dem Polyurethanharnstoff, wie nachstehend näher erläutert, verwendet werden, so bezieht sich die Funktionalität auf einen mittleren Wert, welcher sich durch die Gesamtheit der Makropolyole bzw. Polyole ergibt.

Das zahlenmittlere Molekulargewicht der erfindungsgemäß bevorzugten Polyurethanharnstoffe beträgt vorzugsweise 1000 bis 200000, besonders bevorzugt von 5000 bis 100000. Dabei wird das zahlenmittlere Molekulargewicht gegen Polystyrol als Standard in Dimethylacetamid bei 30 °C gemessen.

### Polyurethanharnstoffe

Im Folgenden werden die erfindungsgemäßen Zusammensetzungen auf Basis von Polyurethanharnstoffen näher beschrieben.

Die erfindungsgemäß vorgesehenen Polyurethanharnstoffe werden im Allgemeinen durch Umsetzung von mindestens einer Makropolyolkomponente, mindestens einer Polyisoyanatkomponente, mindestens einem Polyoxyalkylenether, mindestens einem Diamin und/oder einem Aminoalkohol und gegebenenfalls einer Polyolkomponente gebildet. Weitere Aufbaukomponenten können in dem erfindungsgemäßen Polyurethanharnstoff zugegen sein.

### (a) Makropolyolkomponente

Die Zusammensetzung des erfindungsgemäß vorgesehenen Polyurethanharnstoffes weist Einheiten auf, welche auf mindestens eine Makropolyolkomponente als Aufbaukomponente zurückgehen.

Die Makropolyolkomponente wird dabei im Allgemeinen ausgewählt aus der Gruppe, bestehend aus einem Polyetherpolyol, einem Polycarbonatpolyol, einem Polyesterpolyol und beliebigen Mischungen davon.

In einer bevorzugten Ausführungsform der vorliegenden Erfindung wird die Aufbaukomponente eines aus einem Polyetherpolyol oder einem Polycarbonatpolyol sowie aus Mischungen aus einem Polyetherpolyol und einem Polycarbonatpolyol gebildet.

In einer weiteren Ausführungsform der vorliegenden Erfindung wird die Aufbaukomponente eines Makropolyols aus einem Polyetherpolyol, insbesondere einem Polyetherdiol, gebildet. Polyetherpolyole und insbesondere Polyetherdiole sind hinsichtlich der Freisetzung von Silber besonders bevorzugt. Entsprechende erfindungsgemäße Versuche, welche diese Erkenntnisse stützen, sind weiter unten dargestellt.

Im Folgenden werden die einzelnen Makropolyolaufbaukomponenten näher beschrieben, wobei im Rahmen der vorliegenden Erfindung Polyurethanharnstoffe umfasst sind, welche sowohl nur eine Aufbaukomponente, ausgewählt aus im Allgemeinen Polyetherpolyolen, Polyesterpolyolen und Polycarbonatpolyolen, als auch Mischungen dieser Aufbaukomponenten umfassen. Ferner können die erfindungsgemäß vorgesehenen Polyurethanharnstoffe auch einen oder mehrere unterschiedliche Vertreter dieser Klassen an Aufbaukomponenten umfassen.

Die zuvor definierte Funktionalität der erfindungsgemäß vorgesehenen Polyurethanharnstoffe wird, wenn mehrere unterschiedliche Makropolyole und Polyole oder Polyamine (welche weiter unten unter c) und e) beschrieben sind), im dem Polyurethanharnstoff zugegen sind, als die mittlere Funktionalität verstanden.

### Polyetherpolyol

Die in Frage kommenden, Hydroxylgruppen aufweisenden Polyether sind solche, die durch Polymerisation von cyclischen Ethern wie Ethylenoxid, Propylenoxid, Butylenoxid, Tetrahydrofuran, Styroloxid oder Epichlorhydrin mit sich selbst, z. B. in Gegenwart von BF₃ oder basischen Katalysatoren, oder durch Anlagerung dieser Ringverbindungen, gegebenenfalls im Gemisch oder nacheinander, an Startkomponenten mit reaktionsfähigen Wasserstoffatomen wie Alkoholen und Aminen oder Aminoalkoholen, z. B. Wasser, Ethylenglykol, Propylenglykol-1,2 oder Propylenglykol-1,3, hergestellt werden.

Bevorzugte hydroxylgruppenhaltige Polyether sind solche auf Basis von Ethylenoxid, Propylenoxid oder Tetrahydrofuran oder Mischungen dieser cyclischen Ether. Ganz besonders bevorzugte hydroxylgruppenhaltige Polyether sind solche auf Basis von polymerisiertem Tetrahydrofuran. Es können noch andere hydroxylgruppenhaltige Polyether wie auf Basis Ethylenoxid oder Propylenoxid zugegeben werden, wobei dann aber die Polyether auf Basis von Tetrahydrofuran mindestens zu vorzugsweise 50 Gew.-% enthalten.

### Polycarbonatpolyol

Als Hydroxylgruppen aufweisende Polycarbonate kommen Polycarbonate des durch OH-Zahl bestimmten Molekulargewichts von vorzugsweise 400 bis 6000 g/mol, besonders bevorzugt 500 bis 5000 g/mol, insbesondere von 600 bis 3000 g/mol in Frage, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Bevorzugt enthält die Diolkomponente 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z. B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate auf Basis von Hexandiol-1,6, sowie modifizierend wirkenden Co-Diolen wie z. B. Butandiol-1,4 oder auch von ε-Caprolacton. Weitere bevorzugte Polycarbonatdiole sind solche auf Basis von Mischungen aus Hexandiol-1,6 und Butandiol-1,4.

Das Polycarbonat ist vorzugsweise im Wesentlichen linear ausgebildet und weist nur eine geringfügige dreidimensionale Vernetzung auf, so dass Polyurethanharnstoffe gebildet werden, welche die zuvor genannte Spezifikation aufweisen.

### Polyesterpolyol

Die in Frage kommenden Hydroxylgruppen aufweisenden Polyester sind beispielsweise Umsetzungsprodukte von mehrwertigen, vorzugsweise zweiwertigen Alkoholen mit mehrwertigen, vorzugsweise zweiwertigen Polycarbonsäuren. Anstelle der freien Carbonsäuren können auch die entsprechenden Polycarbonsäureanhydride oder entsprechende Polycarbonsäureester von niedrigen Alkoholen oder deren Gemische zur Herstellung der Polyester verwendet werden.

Die Polycarbonsäuren können aliphatischer, cycloaliphatischer, aromatischer und/oder heterocyclischer Natur sein und gegebenenfalls, z. B. durch Halogenatome, substituiert sein und/oder ungesättigt sein. Bevorzugt sind aliphatische und cycloaliphatische Dicarbonsäuren. Als Beispiele hierfür seien genannt:
Bernsteinsäure, Adipinsäure, Azelainsäure, Sebacinsäure, Phthalsäure, Tetrachlorphthalsäure, Isophtalsäure, Terephthalsäure, Tetrahydrophthalsäure, Hexahydrophthalsäure, Cyclohexandicarbonsäure, Itaconsäure, Sebacinsäure, Glutarsäure, Korksäure, 2-Methylbernsteinsäure, 3,3-Diethylglutarsäure, 2,2-Dimethylbernsteinsäure, Maleinsäure, Malonsäure, Fumarsäure oder Terephthalsäuredimethylester. Anhydride dieser Säuren sind ebenfalls brauchbar, soweit sie existieren. Beispiele hierfür sind Maleinsäureanhydrid, Phthalsäureanhydrid, Tetrahydrophthalsäureanhydrid, Glutarsäureanhydrid, Hexahydrophthalsäureanhydrid und Tetrachlorphthalsäureanhydrid.

Als gegebenenfalls in kleinen Mengen mitzuverwendende Polycarbonsäure sei hier Trimellitsäure genannt.

Als mehrwertige Alkohole kommen vorzugsweise Diole zur Anwendung. Beispiele solcher Diole sind z. B. Ethylenglykol, Propylenglykol-1,2, Propylenglykol-1,3, Butandiol-1,4, Butandiol-2,3, Diethylenglykol, Triethylenglykol, Hexandiol-1,6, Octandiol-1,8, Neopentylglykol, 2-Methyl-1,3-propandiol oder Hydroxypivalinsäureneopentylglykolester in Frage. Auch Polyesterdiole aus Lactonen, z. B. ε-Caprolacton, sind einsetzbar. Als gegebenenfalls mit einzusetzende Polyole sind hier beispielsweise Trimethylolpropan, Glycerin, Erythrit, Pentaerythrit, Trimethylolbenzol oder Trishydroxyethylisocyanurat zu nennen.

### (b) Polyisocyanat

Die erfindungsgemäß vorgesehenen Polyurethanharnstoffe weisen Einheiten auf, welche auf mindestens ein Polyisocyanat als Aufbaukomponente zurückgehen.
Als Polyisocyanate (b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente (b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodekantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente (b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 4,4'-Bis(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion-und/oder Oxadiazintrionstruktur mit mehr als zwei NCO-Gruppen.

Die Menge an Bestandteil (b) in dem erfindungsgemäß vorgesehenen Polyurethanharnstoff beträgt vorzugsweise 1,0 bis 3,5 mol, besonders bevorzugt 1,0 bis 3,3 mol, insbesondere 1,0 bis 3,0 mol, jeweils bezogen auf den Bestandteil (a) des Polyurethanharnstoffs.

### (c) Diamin oder Aminoalkohol

Der erfindungsgemäß vorgesehene Polyurethanharnstoff weist Einheiten auf, welche auf mindestens ein Diamin oder ein Aminoalkohol als Aufbaukomponente zurückgehen und als sogenannte Kettenverlängerer (c) dienen.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Hydrazin, Adipinsäuredihydrazid, 1,4-Bis(aminomethyl)cyclohexan, 4,4'-Diamino-3,3'-dimethyldicyclohexylmethan und andere (C₁ - C₄)-Di- und Tetraalkyldicyclohexylmethan, z. B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-ethylaminopropan, 3-Amino-1-cyclohexylaminopropan, 3-Amino-1- Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil (c) des erfindungsgemäß vorgesehenen Polyurethanharnstoffes kann bei dessen Herstellung als Kettenverlängerer eingesetzt werden.

Die Menge an Bestandteil (c) in dem erfindungsgemäß vorgesehenen Polyurethanharnstoff beträgt vorzugsweise 0,1 bis 1,5 mol, besonders bevorzugt 0,2 bis 1,3 mol, insbesondere 0,3 bis 1,2 mol, jeweils bezogen auf den Bestandteil (a) des Polyurethanharnstoffes.

### (d) Polyoxyalkylenether

Der in der vorliegenden Erfindung vorgesehene Polyurethanharnstoff weist Einheiten auf, welche auf einen Polyoxyalkylenether als Aufbaukomponente zurückgehen.

Bei dem Polyoxyalkylenether handelt es sich vorzugsweise um ein Copolymer aus Polyethylenoxid und Polypropylenoxid. Diese Copolymereinheiten liegen als Endgruppen in dem Polyurethanharnstoff vor und bewirken eine Hydrophilierung des Polyurethanharnstoffes.

Geeignete nichtionisch hydrophilierende Verbindungen entsprechend der Definition der Komponente (d) sind z. B. Polyoxyalkylenether, die mindestens eine Hydroxy- oder Aminogruppe enthalten. Diese Polyether enthalten im Allgemeinen einen Anteil von 30 Gew.-% bis 100 Gew.-% an Bausteinen, die vom Ethylenoxid abgeleitet sind.

Nichtionisch hydrophilierende Verbindungen (d) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylenoxideinheiten zu mindestens 30 mol-%, bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Wenn die Alkylenoxide Ethylenoxid und Propylenoxid eingesetzt werden, können sie in beliebiger Reihenfolge oder auch im Gemisch bei der Alkoxylierungsreaktion eingesetzt werden.

Das mittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil (d) in dem erfindungsgemäß vorgesehenen Polyurethanharnstoff beträgt vorzugsweise 0,01 bis 0,5 mol, besonders bevorzugt 0,02 bis 0,4 mol, insbesondere 0,04 bis 0,3 mol, jeweils bezogen auf den Bestandteil (a) des Polyurethanharnstoffes.

### (e) Polyole

In einer weiteren Ausführungsform umfasst der erfindungsgemäß vorgesehene Polyurethanharnstoff zusätzlich Einheiten, welche auf mindestens ein Polyol als Aufbaukomponente zurückgehen. Bei diesen Polyolaufbaukomponenten handelt es sich im Vergleich zu dem Makropolyol um relativ kurzkettige Aufbaukomponenten, die eine Versteifung durch zusätzliche Hartsegmente hervorrufen können.

Die zum Aufbau der Polyurethanharnstoffe eingesetzten niedermolekularen Polyole (e) bewirken in der Regel daher eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z. B. α-Hydroxybuty-ε-hydroxy-capronsäureester, ω-Hydroxyhexyl-γhydroxybuttersäure-ester, Adipinsäure-(β-hydroxyethyl)ester oder Terephthalsäure-bis(β-hydroxyethyl)-ester können verwendet werden.

Die Menge an Bestandteil (e) in dem erfindungsgemäß vorgesehenen Polyurethanharnstoff beträgt vorzugsweise 0,05 bis 1,0 mol, besonders bevorzugt 0,05 bis 0,5 mol, insbesondere 0,1 bis 0,5 mol, jeweils bezogen auf den Bestandteil (a) des Polyurethanharnstoffes.

### (f) Weitere amin- und/oder hydroxyenthaltende Bausteine (Aufbaukomponente)

Die Umsetzung der isocyanathaltigen Komponente (b) mit den hydroxy- oder aminfunktionellen Verbindungen (a), (c), (d) und gegebenenfalls (e) erfolgt üblicherweise unter Einhaltung eines leichten NCO-Überschusses gegenüber den reaktiven Hydroxy- oder Aminverbindungen. In diesem Fall verbleiben am Endpunkt der Reaktion durch Erreichen einer Zielviskosität immer noch Reste an aktivem Isocyanat. Diese Reste müssen blockiert werden, damit nicht eine Reaktion mit großen Polymerketten stattfindet. Eine solche Reaktion führt zur dreidimensionalen Vernetzung und Vergelung des Ansatzes. Die Verarbeitung eines solchen Polyurethanharnstoffes ist nur eingeschränkt oder nicht mehr möglich. Üblicherweise enthalten die Ansätze hohe Mengen an Wasser. Wasser lässt innerhalb von mehreren Stunden beim Stehen lassen oder beim Rühren des Ansatzes bei Raumtemperatur die noch verbliebenen Isocyanatgruppen abreagieren.

Will man aber den noch verbleibenden Restisocyanatgehalt schnell blockieren, können die erfindungsgemäß vorgesehenen Polyurethanharnstoffe auch Monomere (f) enthalten, die sich jeweils an den Kettenenden befinden und diese abschließen.

Diese Bausteine leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykolmonobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine (f) im Wesentlichen in dem erfindungsgemäß vorgesehenen Polyurethanharnstoff dazu verwendet werden, den NCO-Überschuss zu vernichten, hängt die erforderliche Menge im Wesentlichen von der Menge des NCO-Überschusses ab und kann nicht allgemein spezifiziert werden.

Bevorzugt wird während der Synthese auf diese Bausteine verzichtet. Noch nicht umgesetztes Isocyanat wird dabei vorzugsweise durch das Dispergierwasser hydrolysiert.

### (g) Weitere Bestandteile der erfindungsgemäßen Polyurethanharnstoff-Dispersion

Obwohl die erfindungsgemäßen Polyurethanharnstoff-Dispersion aufgrund der antibakteriellen (antimikrobiellen) Verwendung des silberhaltigen Bestandteils bereits ausreichend funktionalisiert sind, kann es im Einzelfall von Vorteil sein, weitere Funktionalisierungen in die Polyurethanharnstoff-Dispersion und damit in die daraus resultierenden Beschichtungen zu integrieren. Diese weiteren möglichen Funktionalisierungen werden im Folgenden nun beschrieben.
Die erfindungsgemäßen Polyurethanharnstoff-Dispersionen können darüber hinaus weitere, für den angestrebten Zweck übliche Bestandteile wie Additive und Füllstoffe, enthalten. Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Polyurethanharnstoff-Zusammensetzungen verwendet werden können, sind im Allgemeinen beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele solcher pharmakologischer Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotische Mitteln wie Penicilline, Cephalosprine, Vacomycine, Aminoglycoside, Quinolone, Polymxine, Erythromycine; Tertracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon; und
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

Weitere übliche Zusatzstoffe und Hilfsmittel wie Verdickungsmittel, Griffhilfsmittel, Pigmente, Farbstoffe, Mattierungsmittel, UV-Stabilisatoren, phenolische Antioxidantien, Lichstabilisatoren, Hydrophobierungsmittel und/oder Verlaufshilfsmittel können ebenfalls in den erfindungsgemäßen Polyurethanharnstoff-Zusammensetzungen mitverwendet werden.

### (h) Antimikrobiell wirkendes Silber

Die erfindungsgemäße Polyurethanharnstoff-Dispersion umfasst neben dem Polyurethanharnstoff zumindest einen silberhaltigen Bestandteil.

Unter "einem silberhaltigen Bestandteil" wird im Sinne der vorliegenden Erfindung jede Komponente verstanden, die in der Lage ist, Silber in elementarer oder ionischer Form freizusetzen und damit zu einer antimikrobiellen (bioziden / antibakteriellen) Wirkung führt.

Die biozide Wirkung von Silber beruht auf der Wechselwirkung von Silberionen mit Bakterien. Um eine möglichst große Anzahl von Silberionen aus elementarem Silber erzeugen zu können, ist eine große Oberfläche des Silbers vorteilhaft. Daher werden hauptsächlich für antimikoribielle Anwendungen hochporöse Silberpulver, Silber auf Trägermaterialien oder kolloidale Silber-Sole verwendet werden.

Zur Zeit kommerziell erhältlich sind beispielsweise Ag-lon (Silber in einem Zeolith, Agion, Wakefield, MA, USA), Ionpure® (Ag⁺ in Glas, Ciba Spezialitätenchemie GmbH, Lampertheim, Deutschland), Alphasan® (AgZr-Phosphat, Milliken Chemical, Gent, Belgien), Irgaguard® (Ag in Zeolith/Glas), Hygate® (Silberpulver, Bio-Gate, Nürnberg, Deutschland), NanoSilver® BG (Silber in Suspension) und Nanocid® (Silber auf TiO₂ Pars Nano Nasb Co., Teheran, Iran).

Die Silberpulver werden vorzugsweise aus der Gasphase erhalten, wobei eine Silberschmelze in Helium verdampft wird. Die daraus resultierenden Nanopartikel agglomerieren sofort und werden als hochporöse, gut filtrierbare Pulver erhalten. Der Nachteil diese Pulver besteht allerdings darin, dass sich die Agglomerate nicht mehr in Einzelpartikel dispergieren lassen.

Kolloidale Silber-Dispersionen werden durch Reduktion von Silbersalzen in organischen oder wässrigen Medium erhalten. Die Herstellung ist aufwendiger als die der Silberpulver, bietet aber den Vorteil, dass nicht agglomerierte Nanopartikel erhalten werden. Durch das Einarbeiten von nicht agglomerierten Nanopartikeln in Polyurethanharnstoff-Zusammensetzungen können transparente Filme erzeugt werden.

Für die erfindungsgemäßen, silberhaltigen Polyurethanharnstoff-Zusammensetzungen können beliebige Silberpulver oder kolloidale Silberdispersionen verwendet werden. Eine Vielzahl solcher Silbermaterialien ist kommerziell erhältlich.

Die zur Formulierung der erfindungsgemäßen silberhaltigen wässrigen PolyurethanDispersion bevorzugt verwendeten Silbersole werden aus Ag₂O durch Reduktion mit einem Reduktionsmittel wie wässriger Formaldehydlösung nach vorhergehender Zugabe eines Dispergierhilfsmittels hergestellt. Dazu werden die Ag₂O-Sole zum Beispiel durch schnelles Vermischen von Silbernitratlösung mit NaOH durch schnelles Rühren batchweise oder durch Verwendung eines Mikromischers entsprechend der DE 10 2006 017 696 in einem kontinuierlichen Prozess hergestellt. Anschließend werden die Ag₂O-Nanopartikel mit Formaldehyd im Überschuss in einem Batch-Verfahren reduziert und abschließend durch Zentrifugation oder Membranfiltration, bevorzugt durch Membranfiltration, gereinigt. Besonderes vorteilhaft ist diese Produktionsweise, weil die Menge an auf der Oberfläche der Nanopartikel gebundenen organischen Hilfsmitteln hierbei gering gehalten werden kann. Man erhält eine Silbersoldispersion in Wasser mit einer mittleren Teilchengröße von ungefähr 10 bis 150 nm, besonders bevorzugt 20 bis 100 nm. Diese Silbersoldispersion kann dann mit dem erfindungsgemäßen Poylurethanharnstoff vereinigt werden.

In der erfindungsgemäßen nichtionischen Polyurethanharnstoff-Dispersionen können nanokristalline Silberteilchen mit einer mittleren Größe von 1 bis 1000 nm, bevorzugt 5 bis 500 nm, ganz besonders bevorzugt von 10 bis 250 nm, eingesetzt werden. Die Nanosilberteilchen können in organischen Lösungsmitteln oder Wasser dispergiert sein, bevorzugt in wassermischbaren organischen Lösungsmitteln oder Wasser, ganz besonders bevorzugt in Wasser. Die Herstellung der erfindungsgemäßen nichtionischen Polyurethanharnstoff-Zusammensetzung erfolgt im Allgemeinen durch Zugabe der Silberdispersion zu dem Polyurethanharnstoff und anschließendes Homogenisieren durch Rühren oder Schütteln.

Die Menge an nanokristallinem Silber, bezogen auf die Menge an festem Polymer in der wässrigen Polyurethanharnstoff-Dispersion sowie in den daraus resultierenden Beschichtungen unter der Annahme einer homogenen Zusammensetzung und gerechnet als Ag und Ag⁺, kann variabel eingestellt werden. Übliche Konzentrationen gehen von 0,1 bis 10 Gew.-%, bevorzugt von 0,3 bis 5 Gew.-%, besonders bevorzugt von 0,5 bis 3 Gew.-%.

Der Vorteil der Verwendung der erfindungsgemäßen silberhaltigen nichtionischen Polyurethanharnstoff-Zusammensetzung in der Form einer wässrigen Dispersion zur Herstellung von antimikrobiellen Beschichtungen liegt im Vergleich zu alternativen Verfahren in der sehr leichten Kombinationsfähigkeit der wässrigen Polyurethandispersionen und der wässrigen kolloidalen Silberdispersionen. Unterschiedliche Silberkonzentrationen können nach Bedarf für verschiedene Anwendungen leicht und exakt eingestellt werden. Viele Verfahren des Standes der Technik sind wesentlich aufwändiger und auch in der Dosierung der Silbermenge nicht so exakt wie das Verfahren der Herstellung der erfindungsgemäßen Zusammensetzung. Dieses gilt insbesondere für diejenigen Verfahren, bei welchen Polyurethanpellets vor deren Verarbeitung durch Tränkung mit einem antimikrobiellen Mittel ausgestattet werden.

In einer besonders bevorzugten Ausführungsform liegt das antimikrobiell wirksames Silber in Form hochporöser Silberpulver, Silber auf Trägermaterialien oder in Form kolloidaler Silber-sole vor, wobei bezogen auf das feste Polyurethanharnstoffpolymer in der wässrigen Dispersion bzw. der daraus resultierenden Beschichtung unter der Annahme einer homogenen Zusammensetzung 0,1 bis 10 Gew.-% an Silber enthalten ist.

In einer weiteren besonders bevorzugten Ausführungsform liegt das antimikrobiell wirksame Silber in Form kolloidaler Silbersole in wässrigem Medium oder in wassermischbaren organischen Lösungsmitteln mit einer Partikelgröße von 1 bis 1000 nm vor, wobei bezogen auf das feste Polyurethanharnstoffpolymer 0,3 bis 5 Gew.-% zugegeben wird

In einer weiteren besonders bevorzugten Ausführungsform liegt das antimikrobiell wirksame Silber in Form kolloidaler Silbersole in wässrigem Medium mit einer mittleren Partikelgröße von 1 bis 500 nm, wobei bezogen auf das feste Polyurethanharnstoffpolymer in der wässrigen Dispersion bzw. der daraus resultierenden Beschichtung unter der Annahme einer homogenen Zusammensetzung 0,5 bis 3 Gew.-% zugegeben wird.

### Polyurethanharnstoff-Dispersion

In einer bevorzugten Ausführungsform umfasst die erfindungsgemäße nichtionisch stabilisierte Polyurethanharnstoff-Dispersion einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol; und
d) mindestens einem monofunktionellen Polyoxyalkylenether;
   sowie
h) mindestens einen antimikrobiell wirksamen silberhaltigen Bestandteil.

In einer weiteren Ausführungsform der vorliegenden Erfindung umfasst die erfindungsgemäße nichtionisch stabilisierte Polyurethanharnstoff-Dispersion einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol;
d) mindestens einem monofunktionellen Polyoxyalkylenether; und
e) mindestens einem weiteren Polyol;
   sowie
h) mindestens einen antimikrobiell wirksamen silberhaltigen Bestandteil.

In einer weiteren Ausführungsform der vorliegenden Erfindung umfasst die erfindungsgemäße nichtionisch stabilisierte Polyurethanharnstoff-Dispersion einen Polyurethanharnstoff, welcher zumindest aufgebaut wird aus
a) mindestens einem Makropolyol;
b) mindestens einem Polyisocyanat;
c) mindestens einem Diamin oder einem Aminoalkohol;
d) mindestens einem monofunktionellen Polyoxyalkylenether;
e) mindestens einem Polyol; und
f) mindestens einem amin- oder hydroxylhaltiges Monomer, welches sich an den Polymerkettenenden befindet;
   sowie
h) mindestens einen antimikrobiell wirksamen silberhaltigen Bestandteil.

Erfindungsgemäß besonders bevorzugt sind Polyurethanharnstoff-Dispersionen, die einen Polyurethanharnstoff enthalten, der aufgebaut wird aus
a) mindestens einem Makropolyol mit einem mittleren Molgewicht zwischen 400 g/mol und 6000 g/mol und einer Hydroxylfunktionalität von 1,7 bis 2,3 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,0 bis 3,5 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,1 bis 1,5 mol;
d) mindestens einem monofunktionellen Polyoxyalkylenether oder einer Mischung solcher Polyether mit einem mittleren Molgewicht zwischen 500 g/mol und 5000 g/mol in einer Menge pro Mol des Makropolyols von 0,01 bis 0,5 mol;
e) gegebenenfalls einem oder mehreren kurzkettigen aliphatischen Polyolen mit einem Molgewicht zwischen 62 g/mol und 500 g/mol in einer Menge pro Mol des Makropolyols von 0,05 bis 1 mol; und
f) gegebenenfalls amin- oder OH haltige Bausteine, die sich an den Polymerkettenenden befinden und diese abschließen; sowie
h) mindestens einen antimikrobiell wirksamen silberhaltigen Bestandteil enthalten.

Erfindungsgemäß weiter bevorzugt sind Polyurethanharnstoff-Dispersionen, die einen Polyurethanharnstoff enthalten, der aufgebaut wird aus
a) mindestens einem Makropolyol mit einem mittleren Molgewicht zwischen 500 g/mol und 5000 g/mol und einer Hydroxylfunktionalität von 1,8 bis 2,2 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,0 bis 3,3 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,2 bis 1,3 mol;
d) mindestens einem monofunktionellen Polyoxyalkylenether oder einer Mischung solcher Polyether mit einem mittleren Molgewicht zwischen 1000 g/mol und 4000 g/mol in einer Menge pro Mol des Makropolyols von 0,02 bis 0,4 mol;
e) gegebenenfalls einem oder mehreren kurzkettigen aliphatischen Polyolen mit einem Molgewicht zwischen 62 g/mol und 400 g/mol in einer Menge pro Mol des Makropolyols von 0,05 bis 0,5 mol; und
f) gegebenenfalls amin- oder OH haltige Bausteine, die sich an den Polymerkettenenden befinden und diese abschließen; sowie
h) mindestens einen antimikrobiell wirksamen silberhaltigen Bestandteil enthalten.

Erfindungsgemäß noch weiter bevorzugt sind Polyurethanharnstoff-Dispersionen, die einen Polyurethanharnstoff enthalten, der aufgebaut wird aus
a) mindestens einem Makropolyol mit einem mittleren Molgewicht zwischen 600 g/mol und 3000 g/mol und einer Hydroxylfunktionalität von 1,9 bis 2,1 oder Mischungen solcher Makropolyole;
b) mindestens einem aliphatischen, cycloaliphatischen oder aromatischen Polyisocyanat oder Mischungen solcher Polyisocyanate in einer Menge pro Mol des Makropolyols von 1,0 bis 3,0 mol;
c) mindestens einem aliphatischen oder cycloaliphatischen Diamin oder mindestens ein Aminoalkohol als so genannte Kettenverlängerer oder Mischungen solcher Verbindungen in einer Menge pro Mol des Makropolyols von 0,3 bis 1,2 mol;
d) mindestens einem monofunktionellen Polyoxyalkylenether oder einer Mischung solcher Polyether mit einem mittleren Molgewicht zwischen 1000 g/mol und 3000 g/mol in einer Menge pro Mol des Makropolyols von 0,04 bis 0,3 mol, wobei eine Mischung aus Polyethylenoxid und Polypropylenoxid insbesondere bevorzugt ist; und
e) gegebenenfalls einem oder mehreren kurzkettigen aliphatischen Polyolen mit einem Molgewicht zwischen 62 g/mol und 400 g/mol in einer Menge pro Mol des Makropolyols von 0,1 bis 0,5 mol;
h) mindestens einen antimikrobiell wirksamen silberhaltigen Bestandteil enthalten.

### Verwendung der erfindungsgemäßen Polyurethanharnstoff-Dispersionen

Die erfindungsgemäßen nichtionisch stabilisierten Polyurethanharnstoff-Dispersionen können beispielsweise in der Form einer wässrigen Dispersion für eine Vielzahl an unterschiedlichen Anwendungen eingesetzt werden. Dabei stehen insbesondere Anwendungen zur Herstellung von Beschichtungen im Vordergrund, bei welchen es auf eine antimikrobielle Ausrüstung von Gegenständen allgemeiner Art ankommt. Ganz besonders bevorzugt erfolgt die Verwendung der erfindungsgemäßen nichtionisch stabilisierten Polyurethandispersion-Zusammensetzungen beispielsweise in der Form einer wässrigen Dispersion bei der Herstellung von Beschichtungen auf medizinischen Geräten.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetern; Dilatationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Larnygoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsdrähte, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Beschichtungslösungen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsdrähten, Stents und anderen Implantaten gebildet.

Als Substrat der zu beschichtenden Oberfläche kommen viele Materialien in Frage, wie Metalle, Textilien, Keramiken oder Kunststoffe, wobei die Verwendung von Metallen und Kunststoffen für die Herstellung von medizinischen Geräten bevorzugt ist.

Als Beispiele für Metalle können medizinischer Edelstahl oder Nickel-Titan-Legierungen genannt werden.

Im Falle von Kathetern sind diese bevorzugt aus Kunststoffen wie Polyamid, Blockcopolymere aus Styrol und ungesättigten Verbindungen wie Ethylen, Butylen und Isopren, Polyethylen oder Copolymeren aus Polyethylen und Polypropylen, Silikon, Polyvinylchlorid (PVC) und/oder Polyurethanen gefertigt. Zur besseren Haftung der erfindungsgemäßen Polyurethanharnstoff-Zusammensetzungen kann die Oberfläche des Medizinartikels zuvor einer Oberflächenbehandlung wie der Beschichtung mit einem Haftvermittler unterzogen worden sein.

Weiterer Gegenstand der vorliegenden Erfindung sind daher auch Beschichtungen, welche ausgehend von den zuvor beschriebenen Polyurethanharnstoff-Dispersionen erhalten werden.

### Herstellung der erfindungsgemäßen Polyurethanharnstoffe

Die genannten Aufbaukomponenten (a), (b), (d) und gegebenenfalls (e) werden so umgesetzt, dass zunächst ein harnstoffgruppenfreies, isocyanatfunktionelles Prepolymer hergestellt wird, wobei das Stoffmengenverhältnis von Isocyanatgruppen zu mit Isocyanat reaktiven Gruppen 0,8 bis 3,5, bevorzugt 0,9 bis 3,0 besonders bevorzugt 1,0 bis 2,5 beträgt und anschließend werden die verbliebenen Isocyanat-Gruppen vor, während oder in Wasser nach dem Dispergieren aminofunktionell kettenverlängert oder -terminiert, wobei das Äquivalentverhältnis von isocyanatreaktiven Gruppen der zur Kettenverlängerung eingesetzten Verbindungen zu freien Isocyanat-Gruppen des Prepolymers zwischen 40 bis 150 %, bevorzugt zwischen 50 bis 120 %, besonders bevorzugt zwischen 60 bis 120 %, beträgt.

Die erfindungsgemäßen Polyurethanharnstoffe werden vorzugsweise nach dem sogenannte Aceton-Verfahren als Dispersion hergestellt.

Für die Herstellung der Polyurethanharnstoffe nach diesem Aceton-Verfahren werden üblicherweise die Aufbaukomponenten (a), (d) und gegebenenfalls (e), die keine primären oder sekundären Aminogruppen aufweisen dürfen, und die Polyisocyanatkomponente (b) zur Herstellung eines isocyanatfunktionellen Polyurethan-Prepolymers ganz oder teilweise vorgelegt und gegebenenfalls mit einem mit Wasser mischbaren, aber gegenüber Isocyanatgruppen inerten Lösungsmittel verdünnt und auf Temperaturen im Bereich von 50 bis 120 °C aufgeheizt. Zur Beschleunigung der Isocyanatadditionsreaktion können die in der PolyurethanChemie bekannten Katalysatoren eingesetzt werden, zum Beispiel Dibutyzinndilaurat. Bevorzugt ist die Synthese ohne Katalysator.

Geeignete Lösungsmittel sind die üblichen aliphatischen, ketofunktionellen Lösemittel wie z. B. Aceton, Butanon, die nicht nur zu Beginn der Herstellung, sondern gegebenenfalls in Teilen auch später zugegeben werden können. Bevorzugt sind Aceton und Butanon. Andere Lösemittel, wie z. B. Xylol, Toluol, Cyclohexan, Butylacetat, Methoxypropylacetat der Lösemittel mit Ether- oder Estereinheiten, können ebenfalls eingesetzt und ganz oder teilweise abdestilliert werden oder vollständig in der Dispersion verbleiben.

Anschließend werden die gegebenenfalls zu Beginn der Reaktion noch nicht zugegebenen Bestandteile von (a), (b), (d) und gegebenenfalls (e) zudosiert.

Bei der Herstellung des Polyurethan-Prepolymeren beträgt das Stoffmengenverhältnis von Isocyanatgruppen zu mit Isocyanat reaktiven Gruppen 0,8 bis 3,5, bevorzugt 0,9 bis 3,0, besonders bevorzugt 1,0 bis 2,5.

Die Umsetzung der Komponenten (a), (b), (d) und gegebenenfalls (e) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder Butanon gelöst.

Anschließend werden mögliche NH₂-, NH-funktionelle und/oder OH-funktionelle Komponenten mit den noch verbliebenen Isocyanatgruppen umgesetzt. Diese Kettenverlängerung/- terminierung kann dabei entweder in Lösungsmittel vor dem Dispergieren, während des Dispergierens oder in Wasser nach dem Dispergieren durchgeführt werden. Bevorzugt wird die Kettenverlängerung vor der Dispergierung in Wasser durchgeführt.

Werden zur Kettenverlängerung Verbindungen entsprechend der Definition von (c) mit NH₂-, NH- und/oder OH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung eingesetzten Verbindungen zu freien NCO-Gruppen des Prepolymers liegt zwischen 40 bis 150 %, bevorzugt zwischen 50 bis 120 %, besonders bevorzugt zwischen 60 bis 120 %.

Die aminischen/hydroxyhaltigen Komponenten (c) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist. Wenn Wasser oder organische Lösemittel als Verdünnungsmittel mitverwendet werden, so beträgt der Verdünnungsmittelgehalt bevorzugt 70 bis 95 Gew.-%.

Die Herstellung der Polyurethandispersion aus den Prepolymeren erfolgt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den Prepolymerlösungen gerührt. Bevorzugt wird das Wasser in das gelöste Prepolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Feststoffgehalt der Polyurethandispersion liegt zwischen 20 bis 70 Gew.-%, bevorzugt 20 bis 65 Gew.-%. Für Beschichtungsversuche können diese Dispersionen beliebig mit Wasser verdünnt werden, um die Dicke der Beschichtung variabel einstellen zu können.

Weiterer Gegenstand der vorliegenden Erfindung ist ein Verfahren zur Herstellung der erfindungsgemäßen Polyurethanharnstoff-Dispersion durch Vermischen eines wie zuvor definierten nichtionisch stabilisierten Polyurethanharnstoffs bzw. eines wie vorstehend erhaltenen Polyurethanharnstoffes mit einem wie zuvor definierten silberhaltigen Bestandteil.

Die erfindungsgemäßen Polyurethanharnstoff-Dispersion können, wie bereits erwähnt, zur Beschichtung verschiedener Substrate wie beispielsweise Metall, Kunststoff, Keramik, Papier, Leder oder textile Gewebe verwendet werden. Die Beschichtungen können durch verschiedenen Techniken wie Sprühen, Tauchen, Rakeln, Drucken oder Transferbeschichtung auf alle denkbaren Substrate aufgetragen werden. Bevorzugte Anwendungen dieser Polyurethanharnstoff-Zusammensetzungen finden sich beispielsweise für Oberflächen von medizinischen Geräten und Implantaten, wie bereits zuvor erwähnt.

Die Vorteile der erfindungsgemäßen Polyurethanharnstoff-Dispersionen werden durch Ausführungsbeispiele und entsprechende Vergleichsversuche in den folgenden Beispielen dargelegt.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene Gesamtlösung.

### Verwendete Substanzen und Abkürzungen:

- Desmophen^{®} C2200:: Polycarbonatpolyol, OH-Zahl 56 mg KOH/g, zahlenmittleres Mole-kulargewicht 2000 g/mol (Bayer AG, Leverkusen, DE)
- Desmophen^{®} C1200:: Polycarbonatpolyol, OH-Zahl 56 mg KOH/g, zahlenmittleres Mole-kulargewicht 2000 g/mol (Bayer AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- Polyether LB 25:: (monofunktioneller Polyether auf Ethylenoxid-/Propylenoxidbasis zahlenmittleres Molekulargewicht 2250 g/mol, OH-Zahl 25 mg KOH/g (Bayer AG, Leverkusen, DE)

### Beispiel 1:

Dieses Beispiel beschreibt die Herstellung einer erfindungsgemäßen Polyurethanharnstoff-Dispersion

277,2 g Desmophen® C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 3 h 40 min war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 15 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,5 % und einer mittleren Teilchengröße von 164 nm erhalten.

### Beispiel 2:

Dieses Beispiel beschreibt die Herstellung einer erfindungsgemäßen Polyurethanharnstoff-Dispersion.

277,2 g Desmophen® C 1200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2,5 h war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,4 % und einer mittleren Teilchengröße von 146 nm erhalten.

### Beispiel 3:

Dieses Beispiel beschreibt die Herstellung einer erfindungsgemäßen Polyurethanharnstoff-Dispersion.

277,2 g PolyTHF® 2000, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 18 h war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,7 % und einer mittleren Teilchengröße von 166 nm erhalten.

### Beispiel 4:

Dieses Beispiel beschreibt die Herstellung einer erfindungsgemäßen Polyurethanharnstoff-Dispersion.

269,8 g PolyTHF® 2000, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 100 °C. Nach 17,5 h war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,6 % und einer mittleren Teilchengröße von 107 nm erhalten.

### Beispiel 5:

Dieses Beispiel beschreibt die Herstellung einer erfindungsgemäßen Polyurethanharnstoff-Dispersion.

282,1 g PolyTHF® 2000, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21,5 h war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 37,5 % und einer mittleren Teilchengröße von 195 nm erhalten.

### Beispiel 6:

Es wurden eine 0,054 molare Silbernitratlösung mit einer Mischung aus einer 0,054 molaren Natronlauge und dem Dispergierhilfsmittel Disperbyk® 190 (Hersteller BYK Chemie) (1 g/l) in einem Volumenverhältnis von 1:1 versetzt und 10 min gerührt. Ein braun gefärbtes Ag₂O-Nanosol entstand. Zu dieser Reaktionsmischung wurde unter Rühren eine wässrige 4,6 molare Formaldehyd-Lösung zugesetzt, so dass das molare Verhältnis Ag⁺ zu Reduktionsmittel 1:10 betrug. Diese Mischung wurde auf 60°C erwärmt, 30 min bei dieser Temperatur gehalten und anschließend abgekühlt. Die Partikel wurden mittels Zentrifugation (60 min bei 30000 U/min) aufgereinigt und durch Eintrag von Ultraschall (1 min) in vollentsalztem Wasser redispergiert. Dieser Vorgang wurde zweimal wiederholt. Ein kolloidal-stabiles Sol mit einem Feststoffgehalt von 5 Gew.-% (Silberpartikel und Dispergierhilsmittel) wurde so erhalten. Die Ausbeute beträgt knapp 100 %. Die Silberdispersion enthält nach der Zentrifugation laut Elementaranalyse 3 Gew.-% Disperbyk® 190 bezogen auf den Silbergehalt. Eine Untersuchung mittels Laserkorrelationsspektroskopie ergab einen effektiven Durchmesser der Partikel von 73 nm.

50 ml der Polyurethandispersionen der Beispiele 1 bis 5 wurden mit einer 15,1%igen wässrigen kolloidalen Silberdispersion, dessen Herstellung vorstehend beschrieben ist, versetzt und durch Schütteln homogenisiert. In die Polyurethandispersionen der Beispiele 1 bis 5 wird so viel Silberdispersion zugegeben, dass die Dispersionen bezogen auf den festen Polymergehalt 1 Gew.-% Silber enthalten.

**Tabelle 1: Polyurethandispersionen mit 1 Gew.-% nanokristallinem Silber**

| Beispiel | Produkt |
|---|---|
| 6a | PU-Dispersion des Beispiels 1 mit 1 Gew.-% nanokristallinem Silber |
| 6b | PU-Dispersion des Beispiels 2 mit 1 Gew.-% nanokristallinem Silber |
| 6c | PU-Dispersion des Beispiels 3 mit 1 Gew.-% nanokristallinem Silber |
| 6d | PU-Dispersion des Beispiels 4 mit 1 Gew.-% nanokristallinem Silber |
| 6e | PU-Dispersion des Beispiels 5 mit 1 Gew.-% nanokristallinem Silber |

### Beispiel 7: Ag-Freisetzungsstudie chemisch

Die silberhaltigen Beschichtungen zur Messung der Silberfreisetzung wurden auf Objektträger aus Glas der Größe 25x75 mm mit Hilfe eines Spincoaters (RC5 Gyrset® 5, Karl Süss, Garching, Deutschland) hergestellt. Ein zur Haftverbesserung mit 3-Aminopropyltriethoxysilan behandelter Objektträger wurde hierzu auf dem Probenteller des Spincoaters eingespannt und mit ca. 2,5 - 3 g wässriger unverdünnter Polyurethandispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Von den so erhaltenen Objektträgern werden Stücke von 4,5 cm² hergestellt und dann zur Messung der freigesetzten Silbermengen herangezogen.

Die Objektträgerstücke mit verschiedenen silberhaltigen Polyurethanbeschichtungen der Beispiele 6a bis 6e wurden in Tablettenröhrchen mit 2,5 ml destilliertem Wasser überschichtet und 1 Woche im Brutschrank bei 37 °C gelagert. Das Wasser wurde abgenommen und die vom Film in die Flüssigkeit abgegebene Silbermenge per Atomabsorptionsspektroskopie bestimmt. Die trockenen Filme auf den Glasstücken wurden wieder mit 2,5 ml Wasser überschichtet und weiter bei 37 °C gelagert. Das ganze Verfahren wurde 5 mal wiederholt, so dass Silberfreisetzungen für mehrere Wochen bestimmt werden können.

**Tabelle 2: Silberfreisetzung der Filme in wässrige Umgebung**

| | Beispiel 6a | Beispiel 6b | Beispiel 6c | Beispiel 6d | Beispiel 6e |
|---|---|---|---|---|---|
| ng Ag | 450 | 475 | 1350 | 1475 | 400 |
| (Woche 1) | | | | | |
| ng Ag | 77,5 | 400 | 400 | 625 | 182,5 |
| (Woche 2) | | | | | |
| ng Ag | 35 | 170 | 247,5 | 500 | 185 |
| (Woche 3) | | | | | |
| ng Ag | 12,5 | 82,5 | 230 | 250 | 80 |
| (Woche 4) | | | | | |
| ng Ag | 12,5 | 45 | 1025 | 275 | 60 |
| (Woche 5) | | | | | |

Die Ergebnisse zeigen, dass die Beschichtungen über einen längeren Zeitraum Silber abgeben.

### Beispiel 8:

In Aluminiumbehältern (6,4 cm Durchmesser, 1,3 cm hoch) wurden 2,5 g der silberhaltigen Polyurethandispersionen der Beispiele 6b und 6d eingewogen. Man ließ die wässrige Dispersion zunächst 2 h bei Raumtemperatur eintrocknen und trocknete dann die noch feuchten Polymere 25 h im Trockenschrank bei 50 °C. Die entstandenen Formkörper aus Polyurethan wurden von den Aluminiumschalen gelöst und kleine Plättchen mit 5 mm Durchmesser geschnitten. Diese silberhaltigen Polyurethanplättchen von einer Dicke von 150 µm wurden gegen *Escherichia coli* auf ihre antimikrobielle Wirksamkeit geprüft.

Polyurethanplättchen aus den silberhaltigen Polyurethandispersionen der Beispiele 6b und 6d mit einen Durchmesser von 5 mm wurden in einer Bakteriensuspension von *Escherichia coli* ATCC 25922 auf ihre abtötende Wirkung hin untersucht.

Eine Bakterienkultur von *Escherichia coli* ATCC 25922 wurde hergestellt, indem Kolonien von einer bewachsenen und über Nacht bei 37 °C gewachsenen Agarplatte mit Columbia Agar (Columbia Blutagar Platten, Firma Becton Dickinson, # 254071) entnommen wurden und in 0.9 % iger Kochsalzlösung aufgeschwemmt wurden. Von dieser Lösung wurde ein Aliquot in PBS (PBS pH 7,2, Firma Gibco, #20012) mit 5 % Müller Hinton Medium (Becton Dickinson, #257092) überführt, so dass eine OD₆₀₀ von 0.0001 erreicht wurde. Diese Lösung entspricht einer Keimzahl von 1 x 10⁵ Keime pro ml. Die Keimzahl wurde durch Reihenverdünnung und Ausplatieren der Verdünnungsstufen auf Agarplatten bestimmt. Die Zellzahl wurde in Colony forming units (CFU/ml) angegeben.

Die Polyurethanplättchen aus den silberhaltigen Polyurethandispersionen der Beispiele 6b und 6d mit einen Durchmesser von 5 mm wurden in jeweils ein Loch einer 24 Loch Mikrotiterplatte gelegt. In die Löcher auf die Plättchen wurde jeweils 1 ml der ***E.** coli* ATCC 25922 Suspension mit einer Keimzahl von 1 x 10⁵ Keime pro ml pipettiert und bei 37°C 24 h inkubiert. Direkt nach Versuchsansatz, nach 2, 4, 6 und 24 h wurden je 20 µl pro Vertiefung entnommen und eine Keimzahlbestimmung durchgeführt. Nach 24 h wurden die Probeplättchen entnommen und in eine neue 24 Loch Mikrotiterplatte überführt. Auf die Plättchen wurden erneut je 1 ml einer wie oben hergestellten Bakteriensuspension *von E. coli* ATCC 25922 gegeben, bei 37 °C inkubiert und direkt nach Versuchsansatz und nach 24 h jeweils 20 µl Probe entnommen und die Keimzahl bestimmt. Dies wurde bis zu 10 Tage wiederholt.

**Tabelle 3a: Untersuchung der antibakteriellen Wirkung der Polyurethanbeschichtung des Beispiels 6b**

| | Tag 1 | Tag 2 | Tag 3 | Tag4 | Tag 8 |
|---|---|---|---|---|---|
| Keimzahl/ml | 1,5 x10⁵ | 1,0 x10⁵ | 1,0 x10⁵ | 1.5 x10⁵ | 1.0 x10⁵ |
| (0 h) | | | | | |
| Keimzahl/ml | < 100 | 1,6 x10² | 1,6 x10² | 3,0 x10³ | < 100 |
| (24 h) | | | | | |

**Tabelle 3b: Untersuchung der antibakteriellen Wirkung der Polyurethanbeschichtung des Beispiels 6d**

| | Tag 1 | Tag 2 | Tag 3 | Tag 4 | Tag 8 | Tag 9 | Tag 10 |
|---|---|---|---|---|---|---|---|
| Keimzahl/ml | 1,5x10⁵ | 1,0 x10⁵ | 1,0x10⁵ | 1,5x10⁵ | 1,0x10⁵ | 1,5x10⁵ | 2,0x10⁵ |
| (0 h) | | | | | | | |
| Keimzahl/ml | < 100 | 9 x10² | 9 x10² | 1,4 x10² | < 100 | < 100 | < 100 |
| (24 h) | | | | | | | |

Die Ergebnisse weisen eine antibakterielle Beschichtung von über einer Woche nach. Frisch zugegebene Bakteriensuspension wird immer wieder bis auf sehr geringe Keimzahlen abgetötet.

### Beispiel 9:

Die silberhaltigen Beschichtungen zur Messung der antimikrobiellen Wirksamkeit wurden auf Glasobjektträger der Größe 25x75 mm mit Hilfe eines Spincoaters (RC5 Gyrset 5, Karl Süss, Garching, Deutschland) hergestellt. Ein zur Haftverbesserung mit 3-Aminopropyltriethoxysilan behandelter Objektträger wurde hierzu auf dem Probenteller des Spincoaters eingespannt und mit ca. 2,5 - 3 g wässriger unverdünnter Polyurethandispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt zur Messung der antimikrobiellen Wirksamkeit herangezogen.

Die Prüfung der antimikrobiellen Wirksamkeit wurde nach folgender Vorschrift durchgeführt:

Der Testkeim *E. coli* ATCC 25922 wurde in einer Übernachtkultur auf Columbia Agar (Columbia Blutagar Platten, Firma Becton Dickinson, # 254071) bei 37°C kultiviert. Anschließend wurden Kolonien in PBS (PBS pH 7,2, Firma Gibco, #20012) mit 5 % Müller Hinton Medium (Becton Dickinson, #257092) aufgeschwemmt und eine Zellzahl von ca. 1 x 10⁵ Keime/ml eingestellt ("Keimsuspension"). Das Testmaterial wurde in ein mit 30 ml Keimsuspension befülltes Falconröhrchen überführt und über Nacht bei 37°C bebrütet. Nach 24 h wurden 20 µl der Zellsuspension zur Wachstumskontrolle entnommen. Die Zellzahl wurde durch Reihenverdünnung und Ausplattieren der Verdünnungsstufen auf Agarplatten bestimmt. Die Zellzahl wird als Colony forming units (CFU/ ml) angegeben.

**Tabelle 4a: Antibakterielle Wirkung von Polyurethanbeschichtungen**

| | **Beispiel 6a** | **Beispiel 6b** | **Beispiel 6c** | **Beispiel 6d** | **Beispiel 6e** |
|---|---|---|---|---|---|
| **CFU/ml** | 4 x 10⁵ | 4 x 10⁵ | 4 x 10⁵ | 4 x 10⁵ | 4 x 10⁵ |
| **(0 h)** | | | | | |
| **CFU/ml** | < 100 | < 100 | < 100 | < 100 | < 100 |
| **(24 h)** | | | | | |

Die Beschichtung des Beispiels 6d wurde über 15 Tage lang auf antimikrobielle Wirkung geprüft. Hierzu wurde die obere Vorschrift derart weitergeführt, dass nach Bestimmung der Keimzahl wieder frische Bakteriensuspension bekannter Konzentration auf die Beschichtung gegeben und nach 24 h wieder eine Keimzahl bestimmt wird. An den Tagen 4 - 6 und 9 - 14 wurde der Film in PBS-Puffer gewässert.

**Tabelle 4b: Langzeitwirkung der antibakteriellen Beschichtung des Beispiels 6d**

| | Tag 1 | Tag 2 | Tag 3 | Tag 4 | Tag 8 | Tag 15 |
|---|---|---|---|---|---|---|
| Keimzahl/ml | 4 x10⁵ | 7 x10⁵ | 6 x10⁵ | 4 x10⁵ | 3 x10⁵ | 6x10⁵ |
| (0 h) | | | | | | |
| Keimzahl/ml | < 100 | < 100 | < 100 | < 100 | < 100 | < 100 |
| (24 h) | | | | | | |

### Beispiel 10 - Vergleichsversuch:

### Silberfreisetzung von sulfonathaltigen Dispersionen

Es werden Dispercoll® U 53 und Impranil® DLN verwendet. Beides sind polyesterhaltige, auf aliphatische Diisocyanaten basierende Polyurethanharnstoffe, die durch Sulfonsäuregruppen stabilisiert werden

| | Dispercoll U^{®} 53 | Impranil^{®} DLN |
|---|---|---|
| ng Ag | 248 | 325 |
| (Woche 1) | | |
| ng Ag | 70 | 98 |
| (Woche 2) | | |
| ng Ag | 22 | 122 |
| (Woche 3) | | |
| ng Ag | 23 | 20 |
| (Woche 4) | | |
| ng Ag | 142 | 525 |
| (Woche 5) | | |
| ng Ag | 1100 | 1575 |
| (Woche 6) | | |

Vergleich zu erfindungsgemäßen nichtionischen Dispersionen:
- In der ersten und zweiten Woche erfolgt eine relativ niedrige Silberfreisetzung im Vergleich zu den erfindungsgemäßen nichtionischen Dispersionen.
- Beide Sulfonat-haltigen Beschichtungen werden nach 4 bis 5 Wochen Lagerung hart und spröde. Der starke Anstieg der Silberfreisetzung in Woche 5 und 6 lässt sich durch die Zersetzung der Polymermatrix erklären.

### Beispiel 11 - Vergleichsversuch

Weiterhin kann man in Untersuchungen zur Bakterienadhärenz von E. coli an verschiedenen Polyurethan-Oberflächen erkennen, dass zwei Beschichtungen, welche ausgehend von nichtionischen Dispersionen (Beispiel 2 und 4) erhalten werden, schon ohne den Einsatz von Ag eine relativ niedrige Affinität zu E. coli haben. Der Versuch wurde in Anlehnung an die japanische Norm JIS Z 2801 durchgeführt.

Hierzu wird der Testkeim *E. coli* ATCC 25922 in einer Übernachtkultur auf Columbia Agar bei 37 °C kultiviert. Anschließend wurden einige Kolonien in phosphatgepufferter Kochsalzlösung (PBS) mit 5 % Müller Hinton Medium aufgeschwemmt und eine Zellzahl von ca. 1 x 10⁵ Keime/ml eingestellt. Jeweils 100 µl dieser Suspension wurden mit Hilfe eines 20 x 20 mm großen Parafilms auf dem Testmaterial (hier die Beschichtungen ohne nanokristallines Silber) verteilt, so dass die Fläche gleichmäßig mit Zellsuspension benetzt ist. Anschließend wurde das Testmaterial mit der Bakteriensuspension 6 h bei 37 °C in einer Feuchtekammer inkubiert. Nach 6 h wurden zur Kontrolle 20 µl der Zellsuspension zur Wachstumskontrolle entnommen. Die Zellzahl wurde durch Reihenverdünnung und Ausplattieren der Verdünnungsstufen auf Agarplatten bestimmt. Hierbei wurden nur lebende Zellen ermittelt. Bei allen untersuchten Materialien stellte man ein Wachstum der Keime auf etwa 10⁷ bis 10⁸ Keime/ml fest. Die Testmaterialien ohne den Zusatz von nanokristallinem Silber behindern also nicht das Keimwachstum.

Anschließend wurde der Parafilm vom Testmaterial entnommen und das Testmaterial dreimal mit jeweils 4 ml PBS gewaschen, um freischwimmende Zellen zu entfernen. Danach wurde das Testmaterial in 15 ml PBS überführt und 30 Sekunden im Ultraschallbad beschallt, um die an der Testmaterialoberfläche anhaftenden Bakterien abzulösen. Die Colony Forming Units pro ml werden als log-Stufen ausgegeben.

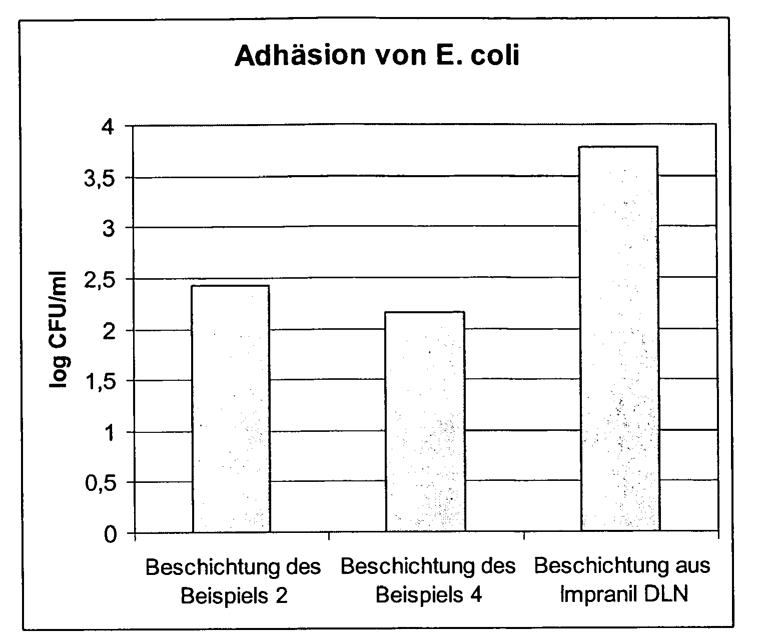

Aus diesem Ergebnis kann abgeleitet werden, dass nichtionische Dispersionen im Vergleich zu einer sulfonathaltigen Dispersion (Impranil DLN) ohne weitere Hilfsmittel eine relativ niedrige Affinität zu E. coli zeigen. Das alleine schützt jedoch noch nicht von Infektion, aber die relativ niedrige Bakterienkonzentration auf der Oberfläche kann dann leicht mit einer relativ niedrigen Silberkonzentration vollständig abgebaut werden.

## Patentansprüche

1. Wässrige Dispersion, umfassend mindestens einen nichtionisch stabilisierten Polyurethanharnstoff und mindestens einen silberhaltigen Bestandteil.

2. Wässrige Dispersion nach Anspruch 1, **dadurch gekennzeichnet, dass** der Polyurethanharnstoff eine Makropolyol-Aufbaukomponente umfasst, die ausgewählt ist aus der Gruppe, bestehend aus mindestens einem Polyesterpolyol, mindestens einem Polyetherpolyol, mindestens einem Polycarbonatpolyol und Mischungen davon.

3. Wässrige Dispersion nach Anspruch 1 oder 2, **dadurch gekennzeichnet, dass** der Polyurethanharnstoff eine Makropolyol-Aufbaukomponente umfasst, die ausgewählt ist aus der Gruppe, bestehend aus mindestens einem Polyetherpolyol, mindestens einem Polycarbonatpolyol und Mischungen davon.

4. Wässrige Dispersion nach einem der Ansprüche 1 bis 3, **dadurch gekennzeichnet, dass** der Polyurethanharnstoff zumindest aufgebaut wird aus den folgenden Aufbaukomponenten:
a) mindestens einem Makropolyol;
b) mindestens einem Polyisocyanat;
c) mindestens einem Diamin oder einem Aminoalkohol;
d) mindestens einem monofunktionellen Polyoxyalkylenether; und
e) gegebenenfalls mindestens einem Polyol.

5. Wässrige Dispersion nach einem der Ansprüche 1 bis 4, **dadurch gekennzeichnet, dass** die Dispersion als silberhaltigen Bestandteil hochporöse Silberpulver, Silber auf Trägermaterialien oder kolloidale Silber-Sole umfasst.

6. Wässrige Dispersion nach einem der Ansprüche 1 bis 5, **dadurch gekennzeichnet, dass** die Dispersion nanokristalline Silberteilchen mit einer mittleren Größe von 1 bis 1000 nm umfasst.

7. Wässrige Dispersion nach einem der Ansprüche 1 bis 6, **dadurch gekennzeichnet, dass** die Menge an Silber, bezogen auf die Menge an festem Polyurethanharnstoffpolymer und gerechnet als Ag und Ag⁺, 0,1 bis 10 Gew.-% beträgt.

8. Verfahren zur Herstellung einer wässrigen Dispersion gemäß einem der Ansprüche 1 bis 7, **gekennzeichnet durch** den Verfahrensschritt des Vermischens mindestens einer nichtionisch stabilisierten Polyurethanharnstoff-Dispersion mit mindestens einem silberhaltigen Bestandteil.

9. Verfahren nach Anspruch 8, **dadurch gekennzeichnet, dass** der Polyurethanharnstoff durch die folgenden Verfahrensschritte erhalten wird:
(I) Vorlegen der Bestandteile (a), (b), (d) und gegebenenfalls (e) und gegebenenfalls Verdünnen mit einem mit Wasser mischbaren, aber gegenüber Isocyanatgruppen inerten Lösungsmittel;
(II) Erwärmen der aus (I) erhältlichen Zusammensetzung auf Temperaturen im Bereich von 50 bis 120 °C;
(III) Zudosieren der gegebenenfalls zu Beginn der Reaktion noch nicht zugegebenen Bestandteile von (a), (b), (d) und (e);
(IV) Auflösen des erhaltenen Prepolymer mit Hilfe von aliphatischen Ketonen; und
(V) Kettenverlängerung des Prepolymers durch Umsetzung mit dem Bestandteil (c).

10. Polyurethanharnstoff-Dispersion, erhältlich nach dem Verfahren gemäß Anspruch 8 oder 9.

11. Verwendung einer Polyurethanharnstoff-Dispersion nach einem der Ansprüche 1 bis 7 oder 10 zur Beschichtung von Oberflächen, insbesondere eines medizinischen Geräts.

12. Beschichtung, erhältlich ausgehend von einer Polyurethanharnstoff-Dispersion nach einem der Ansprüche 1 bis 7 oder 10.
